(19) Europäisches Patentamt
European Patent Office
Office européen des brevets

(11) EP 3 103 436 B1

(12) # FASCICULE DE BREVET EUROPEEN

(45) Date de publication et mention de la délivrance du brevet:
**12.09.2018 Bulletin 2018/37**

(21) Numéro de dépôt: **16173951.1**

(22) Date de dépôt: **10.06.2016**

(51) Int Cl.:
***A61K 8/46*** *(2006.01)* ***A61Q 17/04*** *(2006.01)*
***C07C 317/28*** *(2006.01)* ***C07C 323/41*** *(2006.01)*
***C07C 323/42*** *(2006.01)* ***C07C 317/44*** *(2006.01)*
***C07C 323/60*** *(2006.01)*

(54) **COMPOSÉS DÉRIVÉS DE L'ACIDE 3-(ALKYLTHIO)PROPÉNOÏQUE, ET LEUR APPLICATION EN COSMÉTIQUE**

DERIVATVERBINDUNGEN VON 3-(ALKYLTHIO)PROPENSÄURE, UND IHRE ANWENDUNG IN DER KOSMETIK

COMPOUNDS DERIVED FROM 3-(ALKYLTHIO)PROPENOIC ACID, AND THE USE THEREOF IN COSMETICS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **12.06.2015 FR 1555368**

(43) Date de publication de la demande:
**14.12.2016 Bulletin 2016/50**

(73) Titulaire: **Exsymol**
**98000 Monaco (MC)**

(72) Inventeur: **SEGUIN, Marie-Christine**
**98000 MONACO (MC)**

(74) Mandataire: **Nevant, Marc et al**
**Cabinet Beau de Loménie**
**158, rue de l'Université**
**75340 Paris Cedex 07 (FR)**

(56) Documents cités:
- **TU, S. ET AL: "Chemical constituents and bioactivities of Clinacanthus nutans aerial", MOLECULES, vol. 19, no. 12, 31 décembre 2014 (2014-12-31), pages 20382-20390, XP002755991,**
- **PACHER, T. ET AL: "Alcoholysis of Naturally Occurring Imides: Misleading Interpretation of Antifungal Activities", JOURNAL OF NATURAL PRODUCTS, vol. 73, 8 novembre 2010 (2010-11-08), pages 1389-1393, XP002755992,**
- **CROW, W.D.; GOSNEY, I.: "Isothiazole Chemistry VI. Reactions of Carbanions with N-Ethyl-3-Isothiazolone", AUSTRALIAN JOURNAL OF CHEMISTRY, vol. 22, 31 décembre 1969 (1969-12-31), pages 765-774, XP002755993,**
- **IRÉNA BECK ET AL: "Study of the photochemical behaviour of sunscreens - benzylidene camphor and derivatives", INTERNATIONAL JOURNAL OF COSMETIC SCIENCE, KLUWER ACADEMIC PUBLISHERS, DORDRECHT, NL, vol. 3, 1 janvier 1981 (1981-01-01), pages 139-152, XP001624413, ISSN: 0142-5463**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 103 436 B1

Printed by Jouve, 75001 PARIS (FR)

## Description

La présente invention a pour objet une famille de composés conjugués dérivés de l'acide 3-(alkylthio)propénoïque, ainsi que leurs utilisations en cosmétique comme agent photoprotecteur du rayonnement solaire. L'invention concerne également des compositions cosmétiques destinées à la photoprotection de la peau ou des phanères.

A côté de ses effets bénéfiques (support à la production de vitamine D et de mélatonine, antidépresseur, bien-être, etc), le soleil engendre également au plan cutané des méfaits, à court et long terme : érythème, vieillissement accéléré des tissus, photodermatoses, carcinomes, mélanomes (Ichihashi M. et al., Toxicology, 2003, vol.189, pp.21-39). Et parmi les différentes familles de rayons qui atteignent la surface de la terre, le rayonnement ultraviolet, limité aux radiations ultraviolettes de type A ($\lambda$ 320-400 nm, UV-A) ou B ($\lambda$ 290-320 nm, UV-B), a été identifié comme étant à l'origine de tels méfaits.

La peau dispose toutefois de systèmes intrinsèques de défense qui lui permettent de lutter contre les agressions du soleil. Ainsi, sous l'effet du rayonnement solaire, une photoprotection dite naturelle ou interne s'y exprime déjà, assurée par une production de pigments mélaniques photoprotecteurs, ou encore par un épaississement de la couche cornée avec un plus grand nombre de couches de kératinocytes dans l'épiderme. L'acide trans-urocanique, métabolite de l'histidine, présent en concentration importante dans les couches supérieures de l'épiderme et en particulier dans la couche cornée, participe lui aussi à cette photoprotection naturelle en exprimant un rôle protecteur endogène vis-à-vis de l'action délétère des UV-B (Barresi C. et al., J. Invest. Dermatol., 2011, vol.131, pp.188-194).

De manière extrinsèque, une protection de la peau contre le rayonnement solaire peut être aussi induite (Lacour et al., Annales de Dermatologie et Vénéréologie, 2007, vol.134, pp.18-24). En général, une telle photoprotection, qualifiée d'artificielle par opposition à la photoprotection naturelle, est assurée par des moyens externes comprenant, outre le port de vêtements couvrants, l'application topique de produits cosmétiques photoprotecteurs. Ces produits ou préparations renferment le plus souvent, solubilisées ou dispersées dans un excipient, des substances protectrices que l'on peut classer en trois grandes catégories: les écrans, dont l'action vise à réfléchir l'ensemble des radiations solaires et par conséquent à empêcher leur pénétration dans la peau ; les filtres, dont l'action vise à absorber une partie des radiations solaires, généralement les rayonnements ultraviolets A (320-400 nm) et B (290-320 nm) puis à libérer l'énergie photonique absorbée par échange thermique avec la peau ; et enfin les agents photoprotecteurs dits actifs par opposition aux écrans et filtres, et qui sont en général des substances capables de piéger les espèces réactives de l'oyygène ("EOR" ou "ROS" en anglais) formées lorsque les radiations sont absorbées par des molécules naturellement présentes dans la peau (mécanisme de photo-sensibilisation).

De telles préparations topiques sont en général caractérisées par un facteur de protection solaire ("FPS" en français ou "SPF" en anglais pour "Sun Protection Factor"), indicateur du niveau de protection contre l'érythème solaire (coup de soleil), adapté à des conditions d'ensoleillement et des phototypes de peau particuliers. Pour les formules antisolaires à fort FPS, écrans et filtres sont associés.

Les écrans dans de telles préparations sont la plupart du temps des substances inertes d'origine minérale (poudres : oxyde de zinc, dioxyde de titane, etc). Leur utilisation dans des produits de protection solaire reste toutefois malaisée, avec l'apparition de traces blanches lors de l'application. Une présentation à l'état de nanoparticules améliore l'exploitation de ces écrans, bien que leur sécurité d'emploi sous cette forme est remise en cause depuis quelques années avec un risque potentiel de pénétrer la peau.

Les filtres chimiques organiques sont généralement d'origine synthétique, alternativement d'origine naturelle lorsqu'il s'agit d'huiles ou d'extraits issus d'espèces végétales. Leur efficacité se résume à un effet absorbant de la lumière ultraviolette en raison de la présence structurale de groupements chromophores capables d'absorber puis de dissiper des radiations lumineuses de longueur d'onde déterminée. Les groupements chromophores dans ces filtres chimiques sont quasi-systématiquement des composés porteurs d'un noyau aromatique mono- ou polycyclique (phényl, benzyl, benzylidène, benzoyl, naphtyl, anthracényl, etc) conjugué à des groupements carbonyles ou des systèmes insaturés aliphatiques pour une plus grande délocalisation des électrons sous l'effet des radiations absorbées (état excité), avant retour à un état stable avec dissipation de l'énergie photonique reçue et/ou réémission d'une radiation moins dangereuse (infrarouge par exemple).

Ainsi, l'on peut citer, parmi ces filtres chimiques chromophores appartenant à l'état de la technique (Rai R. et al., Indian J. Dermatol., 2012, vol. 57(5), pp.335-342), disponibles commercialement, les composés suivants dont le spectre d'absorption est plutôt spécifique des UV-B :

- les dérivés esters de l'acide para-aminobenzoïque (PABA) tels le 2-éthylhéxyl-4-diméthylaminobenzoate (padimate O),
- les dérivés de l'acide méthoxycinnamique tels les octyl méthoxycinnamate (octinoxate ou OMC) et 2-éthoxyéthylméthoxycinnamate (cinoxate),
- les dérivés de l'acide salicylique tels les éthylhexylsalicylate (octisalate) et trolamine salicylate,
- le 2-éthylhéxyl-2-cyano-3,3-diphényl-2-propénoate (octocrylène),

- l'acide phényl-benzimidazole sulfonique (ensulizole).

L'on peut citer aussi les composés suivants dont le spectre d'absorption est plutôt spécifique des UV-A :

- les dérivés du dibenzoylméthane tels le butyl méthoxydibenzoylméthane (avobenzone),
- l'acide téréphtalidène dicamphre sulfonique (ecamsule ou Méxoryl SX®).

L'on peut citer enfin les composés suivants dont le spectre d'absorption est plus large (radiations UV-B + UV-A) :

- des benzophénones telles les benzophénone-3 (oxybenzone) et benzophénone-8 (dioxybenzone),
- des dérivés hydroxybenzotriazoles,
- de nouvelles triazines telles les benzoxazines (Brugè et al., Plos One, 2014, 9, e83401).

Pour beaucoup de ces filtres organiques, choisis avant tout pour des critères physico-chimiques (FPS, spectre d'absorption, rémanence à la surface cutanée), il ressort, au-delà de troubles allergiques potentiels (allergies de contact, photo-allergies, démangeaisons) pourtant rapportés ces dernières années (Uter W. et al., Contact Dermatitis, 2014, vol.71, pp.162-9), qu'il n'est pas toujours communiqué sur l'innocuité photochimique de ces agents chromophores, en particulier sur leurs produits de dégradation tels les produits de photo-isomérisation.

Plus récemment, d'autres stratégies de protection ont été développées avec l'introduction dans les formules antisolaires d'agents actifs tels que des enzymes de réparation de l'ADN, et surtout des composés antioxydants tels que les vitamines C et E, ou des polyphénols (Matsui M.S. et al., J. Invest. Dermatol., 2009, vol.14, pp.56-59). L'intérêt pour cette stratégie a été renforcée par la mise en évidence récente de la capacité des rayonnements visible (400-700 nm) et proche infra-rouge (700-1440 nm) à induire la formation de ROS dans la peau (Liebel F. et al., J. Invest. Dermatol., 2012, vol. 132, pp. 1901-1907 ; Schroeder P. et al., Exp. Gerontol., 2008, vol. 43, pp. 629-632). Là encore, l'incorporation de tels ingrédients actifs dans des formules topiques n'est pas sans poser des problèmes : manque de stabilité, coût, et surtout une formation au contact de l'oxygène, ou de ses espèces réactives, de sous-produits dont la toxicologie est souvent inconnue.

En conséquence, prenant en compte ces différents éléments, la demanderesse s'est attachée à identifier de nouveaux composés photoprotecteurs à visée cosmétique topique offrant une protection à large spectre contre le rayonnement solaire, avec comme objectif concomitant de concevoir des produits qui ne forment pas de sous-produits de dégradation ou de photo-conversion pouvant être mal tolérés par la peau. Il est en effet aujourd'hui devenu de première importance dans l'industrie cosmétique ou dermocosmétique d'apporter la preuve de l'innocuité des substances d'intérêt, mais également de l'innocuité des sous-produits réactionnels résultant de l'activité cosmétique visée.

Pour atteindre ces objectifs, la demanderesse s'est originellement intéressée à un composé antioxydant soufré, l'acide 3-méthylthiopropénoique (TMPA), que l'on retrouve dans certaines plantes sous la forme de conjugués, tel le conjugué amide de l'acide t*rans*-3-méthylthiopropénoique (*E*-TMPA) et de l'éthanolamine, isolé dans des graines de plantes légumineuses et pour lequel un potentiel anti-inflammatoire est rapporté (Ikegami F. et al., Chem. Pharm. Bull., 1989, vol.37, pp.1932-1933).

Puis au cours d'une recherche structure-activité visant à optimiser le pouvoir antioxydant de conjugués du TMPA, la demanderesse a découvert qu'un panel de conjugués originaux de type amide obtenus à partir du stéréoisomère non naturel du TMPA, l'acide *cis*-3-méthylthiopropénoïque (*Z*-TMPA), préparé stéréosélectivement (Faria de Medeiros E. et al., J. Chem. Soc. Perkin Trans I, 1991, n°11, pp.27525-2730), présentait une capacité inattendue à absorber aussi efficacement le rayonnement UV-B et à dissiper l'énergie acquise en s'isomérisant pour produire son stéréoisomère de configuration *trans.* Par la suite, la demanderesse a observé que cette particularité s'accompagnait de la propriété de s'opposer à l'isomérisation photo-induite endogène du photoprotecteur naturel évoqué en préambule ci-avant, l'acide *trans*-urocanique. Ceci a pour avantage de préserver l'activité protectrice de ce système intrinsèque de défense de la peau, mais surtout de s'opposer aux effets négatifs liés à la photo-isomérisation de l'acide trans-urocanique. Il est en effet bien établi aujourd'hui que son comportement de "natural sunscreen" (absorption de l'énergie lumineuse) conduit l'acide *trans*-urocanique (ou "*E*-UCA") à se transformer en acide *cis*-urocanique (ou "Z-UCA") pour lequel défavorablement, et venant défaire les bénéfices du E-UCA, est attribué un comportement pro-oxydant responsable de dommages intracellulaires et en particulier de l'ADN (Mc Loone P. et al., J. Invest. Dermatol., 2005, vol.124, pp.1071-1074 ; Gibbs N.K. et al., J. Invest. Dermatol., 2011, vol.131, pp.14-17 et références citées). Le *Z*-UCA est également impliqué défavorablement dans l'effet suppresseur du rayonnement ultraviolet sur le système immunitaire, une telle "immunosuppression UV-induite" se traduisant au niveau cutané par un défaut dans la réponse immune aux agents sensibilisants, une sensibilité accrue aux infections ainsi qu'un risque augmenté de cancers cutanés (Poon T.S.C. et al., J. Invest. Dermatol., 2003, vol.121, pp.184-190). Lutter contre l'immunosuppression induite par les radiations ultra-violettes est d'ailleurs aujourd'hui un autre objectif majeur en photoprotection, et à ce titre a été défini un indice de protection immune ("IPF" en anglais) (Wolf P. et al., J. Invest. Dermatol., 2003, vol.121, pp.1080-1087) que l'on trouve mentionné sur

certaines formules antisolaires.

De manière globale, le panel identifié de conjugués originaux de type amide dérivés de l'acide *cis*-3-méthylthiopropénoïque répond à l'objectif d'apporter en toute sécurité une protection large contre le rayonnement solaire, en raison de :

- un pouvoir antioxydant, qui leur permet de s'opposer au stress oxydatif généré par les réactions de photo-sensibilisation impliquant des chromophores endogènes (porphyrines, quinones, flavines, etc) et des rayonnements aussi divers que les ultraviolets, le visible et le proche infrarouge. Un tel effet est ainsi capable de s'opposer à la formation d'hydroperoxydes (SqOOH) formés par réaction de l'oxygène singulet sur le squalène [cf. test 1 ci-après]. Il s'oppose aussi au stress oxydatif intra-cellulaire induit par la production de "*cis*-UCA" selon de premiers résultats d'étude. Plus largement et d'intérêt dans le contexte d'une innocuité recherchée des sous-produits réactionnels, il est souligné que ce pouvoir anti-oxydant conduit à des formes oxydées du radical thioéther des conjugués selon l'invention, successivement un sulfoxyde et une sulfone, tous deux bien tolérés par les cellules cutanées [cf. test 2 ci-après] ;
- une capacité à améliorer la survie de cellules épidermiques (lignée cellulaire de kératinocytes immortalisés "HaCaT") exposées à des doses cytotoxiques d'UV-B, ceci de façon dose-dépendante et dès l'utilisation de concentrations faibles en substance active [cf. test 3 ci-après] ;
- une capacité à absorber fortement, tel un filtre, le rayonnement ultraviolet B, puis à dissiper l'énergie absorbée en s'isomérisant en un dérivé dépourvu de toxicité [cf. test 4 ci-après], et sans la genèse d'une activité indésirable contrairement à l'acide urocanique par exemple. Un tel comportement trouve de surcroît un intérêt renforcé lorsque le métabolite photo-induit est un composé "nature-identique" du fait de sa présence identifiée à l'état naturel.
- enfin, la propriété de s'opposer à l'isomérisation photo-induite de l'acide trans-urocanique, ce qui permet de limiter *in fine* les effets immunosuppresseurs du rayonnement ultraviolet [cf. test 5 ci-après].

Concernant l'état de la technique, l'art antérieur révèle, à la connaissance de la demanderesse, une même photo-isomérisation avec rétention de l'activité d'intérêt pour des dérivés benzylidène camphre utilisés comme filtre ultraviolet dans des préparations antisolaires (Beck I. et al., Int. J. Cosmetic Science, 1981, vol.3, pp.139-152). Mais ces dérivés du camphre restent structuralement éloignés de ceux objets de la présente invention.

Concernant l'inhibition de la conversion UV-induite de l'acide *trans*-urocanique en acide *cis*-urocanique immunosupresseur, figurent dans l'état de la technique des agents annoncés "immunoprotecteurs", tels les acides phénylbenzimidazole-sulfoniques (Hurks H.M.H et al., J. Invest. Dermatol., 1997, vol.109, pp.699-703), des cinnamates, benzophénones et autres dibenzoylméthanes (Fillay-Jones J.J. et al., Mutation Res., 1998, vol.422, pp.155-159), etc, tous à nouveau structuralement éloignés de ceux objets de la présente invention.

Par ailleurs au-delà de ces aspects, il peut être signalé un art antérieur avec des composés porteurs eux aussi d'un motif thiopropénamide, d'intérêts multiples (brevets US 5464832 et EP0410726 ; brevets US 5300672 et US 3914301). Dans ces derniers documents, les composés visés affichent toutefois distinctement des vertus antimicrobiennes, fongicides, bactéricides et biocides.

L'invention a donc pour premier objet une famille de conjugués *cis* ou (*Z*)-isomères obtenus purs par réaction de couplage entre l'acide (*Z*)-3-(alkylthio)propénoïque et des alkylamines primaires ou secondaires ou encore une sélection d'acides aminés polaires non ionisables et hydroxylés, caractérisée en ce que ladite famille est représentée par la formule générale (I) suivante :

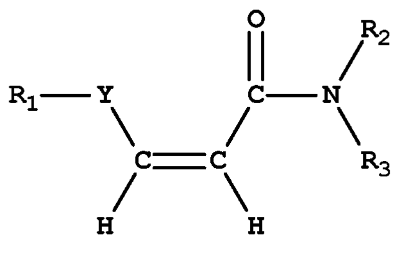

(I)

dans laquelle :

- Y = S ou SO ;
- $R_1$ est un radical alkyle en $C_1$-$C_4$, linéaire ou ramifié ;
- $R_2$ est un atome d'hydrogène ; un radical alkyle en $C_1$-$C_4$, linéaire ou ramifié ;
- $R_3$ est :
  - un radical alkyle en $C_1$-$C_{18}$, un radical alcène en $C_3$-$C_{18}$, un radical alcyne en $C_3$-$C_4$, ledit radical alkyle, alcène

ou alcyne étant linéaire ou ramifié, et optionnellement substitué par un radical hydroxyle ou par un groupe hydroxyphényle ou dihydroxyphényle ;
- la chaîne latérale d'un acide aminé choisi parmi la tyrosine, l'hydroxyproline, la sérine et ses analogues homosérine et isosérine, la thréonine et ses analogues homothréonine et isothréonine.

L'homme du métier n'aura aucun mal à identifier la chaîne latérale des acides aminés susmentionnés. Ces acides aminés sont représentés par l'une des formules suivantes :

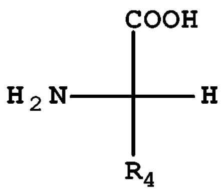

ou

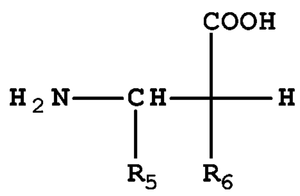

qui correspondent à la tyrosine ($R_4$ = 4-hydroxyphénylméthyle), à la sérine ($R_4$ = $-CH_2-OH$) et ses analogues homosérine ($R_4$ = $-CH_2-CH_2-OH$) et isosérine ($R_5$ = H et $R_6$ = OH), à la thréonine ($R_4$ = $-CH(OH)-CH_3$) et ses analogues homothréonine ($R_4$ = $-CH_2-CH(OH)-CH_3$) et isothréonine ($R_5$ = $CH_3$, $R_6$ = OH). Ces acides aminés peuvent être aussi définis par un numéro d'enregistrement auprès d'une banque de données (hydroxyproline, CAS n° 51-35-4).

Suivant un mode de réalisation préféré de l'invention, les conjugués (*Z*)-isomères selon l'invention sont représentés par la formule (II) ci-après :

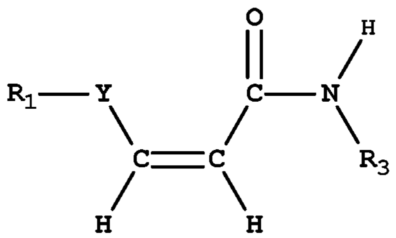

(II)

dans laquelle :

- Y = S ou SO ;
- $R_1$ est un radical alkyle en $C_1$-$C_4$, linéaire ou ramifié ;
- $R_3$ est un radical alkyle en $C_1$-$C_4$, un radical alcène en $C_3$-$C_4$, un radical alcyne en $C_3$-$C_4$, ledit radical alkyle, alcène ou alcyne étant linéaire ou ramifié,

et optionnellement substitué par un radical hydroxyle ou par un groupe hydroxyphényle ou dihydroxyphényle.

Avantageusement, les conjugués (*Z*)-isomères de formule (I) ou (II) sont tels que Y = S.

Avantageusement, les conjugués (*Z*)-isomères de formule (I) ou (II) sont tels que $R_1$ est un radical méthyle.

Avantageusement, les conjugués (*Z*)-isomères de formule (I) ou (II) sont tels que $R_3$ est un radical éthyle, substitué par un radical hydroxyle, un groupe hydroxyphényle ou un groupe dihydroxyphényle. Ainsi à titre d'exemple d'alkylamines engagées dans la réaction de couplage avec l'acide *cis*-3-méthylthiopropénoique (*Z*-TMPA), l'on peut citer l'éthanolamine, la tyramine, une catécholamine telle que la dopamine, l'adrénaline ou la noradrénaline.

A titre d'exemples non limitatifs de conjugués (*Z*)-isomères de formule (I) ou (II), l'on peut citer les composés suivants pour lesquels le produit de photo-isomérisation induit est naturellement présent dans les plantes :

- le (*Z*)-*N*-(2-hydroxyéthyl)-3-(méthylthio)propénamide
- le (*Z*)-*N*-(4-hydroxyphénéthyl)-3-(méthylthio)propénamide.

A ce jour et à la connaissance de la demanderesse, les conjugués (*Z*)-isomères de formule (I) ci-dessus sont nouveaux à l'exception des composés 2-*cis*-entadamide A, isopenangine, N-éthyl-*cis*-3-butylmercaptoacrylamide et N-ethyl-*cis*-3-méthylmercaptoacrylamide.

Les conjugués de formule (I) peuvent être synthétisés selon le procédé d'obtention suivant mis au point par la demanderesse :

i/ préparation stéréosélective de l'acide (*Z*)-3-thiocyanatopropénoique par réaction d'acide propiolique sur le thiocyanate de potassium ;

ii/ réaction d'alkylation à basse température de l'acide (*Z*)-3-thiocyanatopropénoïque selon i/ à l'aide d'iodure d'alkyle, préférentiellement l'iodure de méthyle, et obtention du synthon résultant acide (*Z*)-3-(alkylthio)propénoïque, préférentiellement l'acide (*Z*)-3-méthylthiopropénoïque ;

iii/ réaction de couplage à température ambiante entre l'acide (*Z*)-3-alkylthiopropénoïque selon ii/, préférentiellement l'acide (*Z*)-3-méthylthiopropénoïque, et une alkylamine primaire ou secondaire ou un acide aminé polaire, préférentiellement une alkylamine primaire linéaire ou ramifiée et encore plus préférentiellement l'éthanolamine ou la tyramine, et obtention des dérivés conjugués résultants, préférentiellement les (*Z*)-N-(2-hydroxyéthyl)-3-(méthylthio)propénamide et (*Z*)-*N*-(4-hydroxyphénétyl)-3-(méthylthio)propénamide, le couplage s'effectuant à l'aide d'un agent de couplage bien connu de l'homme de l'art, préférentiellement le N,N'-dicyclohexylcarbodiimide, et d'un activateur de la fonction carboxylique du synthon acide (*Z*)-3-alkylthiopropénoïque, lui aussi bien connu de l'homme de l'art, préférentiellement l'hydroxybenzotriazole.

Ces conjugués (*Z*)-isomères se présentent sous forme solide pulvérulente, soluble en milieu alcoolique, stable aussi bien en poudre qu'en solution sur une durée supérieure à 28 jours.

Les conjugués susmentionnés (*Z*)-*N*-(2-hydroxyéthyl)-3-(méthylthio)propénamide et (*Z*)-*N*-(4-hydroxyphénétyl)-3-(méthylthio)propénamide présentent les caractéristiques physico-chimiques et spectrales suivantes :

- (*Z*)-*N*-(2-hydroxyéthyl)-3-(méthylthio)propénamide

$R_f$ = 0,40 ($CHCl_3$/MeOH, 90:10) ;
p.f. = 112°C ;
$^1H$ RMN ($CD_3OD$) : $\delta$ = 2,32 ppm (s, 3H, $CH_3$), 3,31 (t, 2H, J = 6 Hz, $CH_2$-NH), 3,60 ppm (t, 2H, J = 6 Hz, $CH_2$-OH), 5,89 ppm (d, 1H, J = 10 Hz, CH=), 6,93 ppm (d, 1H, J = 10 Hz, CH=) ;
$^{13}C$ RMN ($CD_3OD$) : $\delta$ = 19,2 ppm ($CH_3$), 42,8 ppm (N-$CH_2$), 61,8 ppm ($CH_2$-O), 115,9 ppm (=CH), 148,6 ppm (=CH-S), 169,2 ppm (C=O) .

- (*Z*)-*N*-(4-hydroxyphénétyl)-3-(méthylthio)propénamide

$R_f$ = 0,35 (Toluène/acétone/acide acétique, 100:50:2) ;
p.f. = 132°C ;
$^1H$ RMN ($CD_3OD$) : $\delta$ = 2,31 ppm (s, 3H, $CH_3$-S), 2,69 (t, 2H, J = 7,5 Hz, $CH_2$-Ph), 3,35 ppm (t, 2H, J = 7,5 Hz, $CH_2$-N), 5,83 ppm (d, 1H, J = 10 Hz, CH=), 6,90 ppm (d, 1H, J = 10 Hz, CH=), 6,70 ppm (d, 2H, J = 8,5 Hz, $C_{Ph}$-H), 7, 02 ppm (d, 2H, J = 8,5 Hz, $C_{Ph}$-H) ;
$^{13}C$ RMN ($CD_3OD$) : $\delta$ = 19,2 ppm ($CH_3$-S), 35,9 ppm ($CH_2$-Ph), 42,2 ppm ($CH_2$-N), 116 ppm (CH=), 116,2 ppm (phénol), 130,7 ppm (phénol), 131,3 ppm (phénol), 148,4 ppm (CH=), 156,9 ppm (phénol), 169 ppm (C=O).

Selon un deuxième aspect, l'invention s'étend également à une composition, à usage cosmétique ou dermocosmétique, destinée à la photoprotection, et en particulier de la peau ou des phanères. Cette composition comprend en association avec tout adjuvant physiologiquement compatible avec la peau ou les phanères, à titre d'ingrédient actif principal, un composé de formule générale (III) ci-dessous :

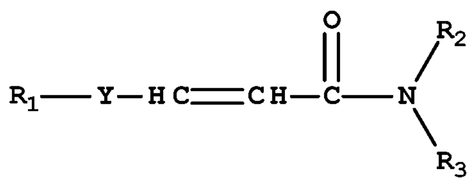

(III)

dans laquelle :

- Y, $R_1$, $R_2$ et $R_3$ sont tels que définis ci-dessus pour les composés de formule (I).

De manière avantageuse, la composition cosmétique ou dermocosmétique selon l'invention comprend, à titre d'ingrédient actif principal, un composé de formule (IV) :

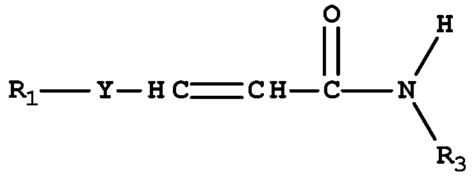

(IV)

dans laquelle :

- Y = S ou SO ;
- $R_1$ est un radical alkyle en $C_1$-$C_4$, linéaire ou ramifié ;
- $R_3$ est un radical alkyle en $C_1$-$C_4$, linéaire ou ramifié, optionnellement substitué par un radical hydroxyle ou par un groupe hydroxyphényle ou dihydroxyphényle.

La formule générale (III) englobe à la fois les isomères Z de formule (IIIa), les isomères E de formule (IIIb), et les mélanges, de préférence racémiques, de ces isomères :

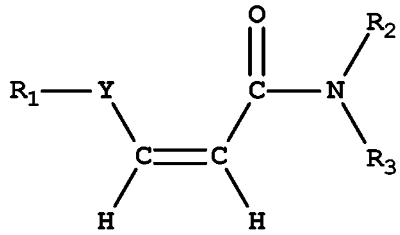

(IIIa)

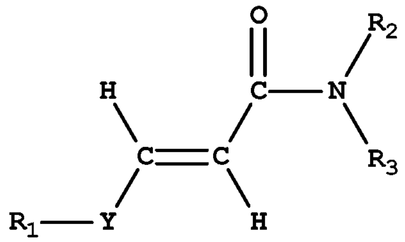

(IIIb)

.

De la même manière, la formule générale (IV) englobe à la fois les isomères Z de formule (IVa), les isomères E de formule (IVb), et les mélanges, de préférence racémiques, de ces isomères :

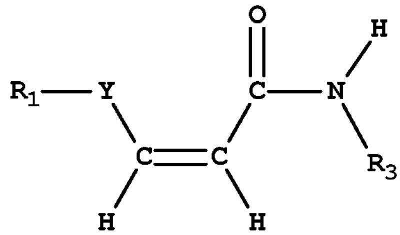

(IVa)

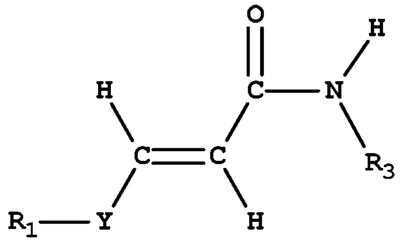

(IVb)

.

Dans le cadre de la présente invention, on entend par "ingrédient actif principal" une substance active capable de s'opposer aux effets délétères du rayonnement solaire.

Avantageusement, la quantité de composé de formule générale (III) ou (IV) dans les compositions visées est comprise entre 0,001 % et 0,1 % en poids par rapport au poids total de la composition, de préférence entre 0,005 % et 0,05 % en poids.

En terme de concentration molaire recommandée en composé de formule générale (III) ou (IV) dans les compositions visées, celle-ci est avantageusement comprise entre 0,05 et 5 mM, de préférence entre 0,25 et 2,5 mM tel qu'illustré par les tests ci-après.

Avantageusement, le composé de formule générale (III) ou (IV) est tel que Y = S.

Avantageusement, le composé de formule générale (III) ou (IV) est tel que $R_1$ est un radical méthyle.

Avantageusement, le composé de formule générale (III) ou (IV) est tel que $R_3$ est un radical éthyle, substitué par un radical hydroxyle ou par un groupe hydroxyphényle ou dihydroxyphényle. Ainsi à titre d'exemple d'alkylamines primaires ou secondaires correspondantes engagées dans la réaction de couplage avec les acides *Z*-TMPA et *E*-TMPA, l'on peut citer l'éthanolamine, la tyramine, une catécholamine telle que la dopamine, l'adrénaline ou la noradrénaline.

Tout particulièrement dans les compositions selon l'invention, le composé de formule générale (III) ou (IV) est un conjugué (*Z*)-isomère de formule (IIIa) ou, respectivement, (IVa) ci-avant dérivé de l'acide (*Z*)-3-(alkylthio)propénoïque.

De manière particulièrement avantageuse, les compositions de l'invention comprennent, à titre d'ingrédient principal, le (*Z*)-*N*-(2-hydroxyéthyl)-3-(méthylthio)propénamide ou le (*Z*)-*N*-(4-hydroxyphénéthyl)-3-(méthylthio)propénamide.

Les compositions selon l'invention sont adaptées à une administration par voie topique cutanée, présentées sous toutes les formes normalement utilisées pour une telle administration. A titre indicatif et non limitatif, les compositions peuvent se présenter sous la forme de suspensions, lotions, crèmes, gels aqueux ou hydroalcooliques, poudres et émulsions multiples pouvant être éventuellement des microémulsions ou des nanoémulsions, etc.

Les compositions selon l'invention peuvent contenir comme adjuvant physiologiquement acceptable au moins un adjuvant connu de l'homme du métier et acceptable dans les domaines cosmétique ou dermocosmétique, choisi parmi les huiles, les cires, les élastomères de silicone, les tensioactifs, les co-tensioactifs, les épaississants et/ou gélifiants, les humectants, les émollients, les filtres solaires organiques ou inorganiques, les photostabilisants, les conservateurs à l'exception des conservateurs donneurs d'aldéhydes, les colorants, les agents matifiants, les agents tenseurs, les séquestrants, les parfums, etc, et leurs mélanges.

Lesdits filtres solaires organiques ou inorganiques sont actifs dans l'UV-A et/ou l'UV-B, choisis parmi les anthranilates, les dérivés cinnamiques, les dérivés de dibenzoylméthane, les dérivés salicyliques, les dérivés du camphre, les dérivés de triazine, les dérivés de la benzophénone, les dérivés de beta,beta-diphénylacrylate, les dérivés de benzotriazole, les dérivés de benzalmalonate, les dérivés de benzimidazole, les imidazolines, les dérivés bis-benzoazolyle, les dérivés de benzoxazole, les dérivés de l'acide p-aminobenzoïque (PABA), les dérivés de méthylène bis-(hydroxyphényl benzotriazole), les polymères filtres et silicones filtres, les dimères dérivés d'alpha-alkylstyrène, les 4,4-diarylbutadiènes, des pigments ou des nanopigments d'oxydes métalliques (oxydes de titane, de zinc, de fer, de zirconium, de cérium), enrobés ou non, et leurs mélanges.

Les compositions selon l'invention peuvent en outre comprendre un ou plusieurs actifs additionnels, l'homme du métier veillant toutefois à ce que les éventuels composés complémentaires actifs, ainsi que leurs proportions, soient choisis de telle manière à ce que les propriétés avantageuses reconnues aux compositions selon l'invention ne soient pas altérées. Ces actifs additionnels peuvent être choisis, sans que cette liste ne soit limitative, parmi les agents déglycants, les agents stimulant la synthèse de collagène ou d'élastine ou prévenant leur dégradation, les agents stimulant la synthèse de glycosaminoglycanes ou de protéoglycanes ou prévenant leur dégradation, les agents augmentant la prolifération cellulaire, les agents dépigmentants ou pro-pigmentants, les agents antioxydants ou anti-radicalaires ou anti-pollution, les agents hydratants, les agents stimulant la lipolyse, les agents drainants ou détoxifiants, les agents anti-inflammatoires, les agents accélérateurs de pénétration, les agents desquamants, les agents apaisants et/ou anti-irritants, les agents astringents, les agents agissant sur la microcirculation, etc, et leurs mélanges.

Les compositions selon l'invention peuvent aussi comprendre en outre au moins un agent de bronzage et/ou de brunissage de la peau.

Un autre objet de l'invention concerne l'utilisation, dans une composition cosmétique ou dermocosmétique, d'un composé de formule (III) ou (IV) tel que défini ci-dessus comme agent photoprotecteur du rayonnement solaire.

Un autre objet de l'invention concerne un composé de formule (III) ou de formule (IV) tel que défini ci-dessus, ou un mélange de ces composés, pour son utilisation, dans une composition cosmétique ou dermocosmétique, pour la photoprotection contre le rayonnement solaire.

Un autre objet de l'invention concerne une méthode de photoprotection contre le rayonnement solaire qui comprend l'application d'une composition cosmétique ou dermocosmétique telle que définie ci-dessus, contenant un composé de formule (III) ou (IV).

De préférence dans les utilisations et la méthode ci-dessus, le composé de formule (III) ou (IV) est un conjugué (Z)-isomère de formule (IIIa) ou, respectivement, (IVa) ci-avant dérivé de l'acide (Z)-3-(alkylthio)propénoïque, en particulier le (*Z*)-*N*-(2-hydroxyéthyl)-3-(méthylthio)propénamide ou le (*Z*)-*N*-(4-hydroxyphénéthyl)-3-(méthylthio)propénamide.

Exemple 1 :

A titre d'illustration, il est mentionné ci-après quatre exemples de formulation de composition selon l'invention, contenant un conjugué dérivé de l'acide *cis*-3-méthylthiopropénoique de formule générale (I) précitée :

*Formule A (crème)*

| | |
|---|---|
| (*Z*)-*N*-(2-hydroxyéthyl)-3-(méthylthio)propénamide | 0,008% |
| Polyisobutène hydrogéné | 7 % |
| Myristate d'isobutyle | 3 % |
| Palmitate de cétyle | 7 % |
| Monostéarate d'éthylène glycol | 5 % |
| Laurate sorbitan | 2 % |
| Polysorbate 20 | 2 % |
| Carbomer (copolymère d'acrylate/acrylamide & huile minérale) | 0,3 % |
| Phénoxyéthanol | 0,5 % |
| Eau | qsp 100% |

*Formule B (gel)*

| | |
|---|---|
| (*Z*)-*N*-(4-hydroxyphénéthyl)-3-(méthylthio)propénamide | 0,011 % |
| Carbomer (copolymère d'acrylate/acrylamide & huile minérale) | 1,5 % |
| Benzoate de sodium | 0,2 % |
| Acide sorbique | 1 % |
| 1,3-butanediol | 10 % |
| Glycérine | 5 % |
| Soude | 0,13 % |
| Phénoxyéthanol | 0,9 % |
| Eau | qsp 100% |

*Formule C (lotion)*

| | |
|---|---|
| (*E*)-*N*-(4-hydroxyphénéthyl)-3-(méthylthio)propénamide | 0,031 % |
| Chlorphénésine | 0,2 % |
| Phenonip (parabènes - parahydroxybenzoate de butyle, éthyle, isobutyle, méthyle, propyle et phénoxyéthanol) | 0,6 % |
| Gomme xanthique | 0,3 % |
| Glycérine | 2,5 % |
| Triéthanolamine | 0,03 % |
| Eau | qsp 100% |

*Formule D (émulsion)*

| | |
|---|---|
| (*Z*)-*N*-(2-hydroxyéthyl)-3-(méthylthio)propénamide | 0,018% |
| Polydécène hydrogéné | 8 % |
| Triglycérides capriques/capriliques | 2 % |
| Oléate d'éthoxydiglycol | 8 % |
| Stéarate de glycéryle | 2 % |
| Diméthicone | 1 % |

(suite)

| | |
|---|---|
| Stéarate de polyéthylène glycol-100 et stéarate de glycéryle | 5 % |
| Parabène de propyle | 0,3 % |
| Alcool stéarylique | 1 % |
| EDTA (éthylènediamine-sel disodique dihydraté de l'acide tétraacétique) | 0,2 % |
| Glycérine | 3 % |
| Gomme xanthique | 0,4 % |
| Huile de germe de blé | 1 % |
| Huile de graines de Macadamia | 1 % |
| Polyéthylène glycol-8 & tocophérol & palmitate d'ascorbyle & acide ascorbique & acide citrique | 0,07 % |
| Triéthanolamine | 0,35 % |
| Eau | qsp 100% |

(Dans tous ces exemples les % sont des % en poids).

Exemple 2 :

L'invention est illustrée ci-après, à titre purement indicatif, par les tests suivants évoqués plus haut dans la description de l'invention (tests 1 à 5).

Test 1 : mise en évidence du potentiel antioxydant des composés de formule (IIIa) et (IIIb) à l'encontre de l'hydroperoxydation du squalène par l'oxygène singulet

Principe : il s'agit de mettre en évidence un éventuel effet de piégeage ("quenching") des composés selon l'invention vis-à-vis d'hydroperoxydes de squalène générés par l'action de l'oxygène moléculaire dissous sur le squalène en présence d'un photosensibilisant (bleu de méthylène).

Expérimentalement, une solution de squalène à 5g/100 mL, une solution de bleu de méthylène à 5 mM et une solution de composé selon l'invention (de 1 à 5 mM) ou de composé témoin (méthylthiopropylamido acétyl méthionine - AMDM) ont été préparées après dissolution dans 100 mL de méthanol (qualité HPLC). Ensuite dans un four à UV-B, chaque solution a été versée à parts égales dans une boîte de Pétri, sans couvercle, afin d'être soumise à une dose d'irradiation (1D) de 1,42 $J.cm^{-2}$. En fin d'irradiation, les volumes de solution ont été récupérés, ajustés à 20 mL avec du méthanol avant analyse par dosage HPLC et après élimination du bleu de méthylène. Le taux de "quenching" a alors été déduit de l'équation suivante :

```
                qté  squalène  oxydé  (témoin)  –  qté  squalène
                oxydé (test)
% protection = --------------------------------------------
                qté de squalène oxydé (témoin) x 100
```

Les résultats obtenus sont rassemblés dans le tableau 1 suivant :

Tableau 1

| **Composé** | **% protection du squalène** |
|---|---|
| Témoin (AMDM, 1mM) | 3 |
| Témoin (AMDM, 5mM) | 15 |
| (*Z*)-*N*-(2-hydroxyéthyl)-3-(méthylthio)propénamide (1mM) | 24 |
| (*Z*)-*N*-(2-hydroxyéthyl)-3-(méthylthio)propénamide (5mM) | 25 |
| (*Z*)-*N*-(4-hydroxyphénéthyl)-3-(méthylthio)propénamide (1mM) | 35 |
| (*Z*)-*N*-(4-hydroxyphénéthyl)-3-(méthylthio)propénamide (5mM) | 44 |
| (*E*)-*N*-(2-hydroxyéthyl)-3-(méthylthio)propénamide (1mM) | 21 |

(suite)

| Composé | % protection du squalène |
|---|---|
| (*E*)-*N*-(4-hydroxyphénéthyl)-3-(méthylthio)propénamide (1mM) | 30 |

Les résultats du tableau 1 soulignent que les composés selon l'invention sont capables de s'opposer efficacement à l'oxydation photo-induite du squalène en hydroperoxydes.

Test 2 : mise en évidence de l'absence de potentiel cytotoxique des sous-produits d'oxydation de conjugués (Z)-isomères de formule (I)

Expérimentalement, l'essai a été réalisé sur une lignée de kératinocytes immortalisés "HaCaT", maintenue par repiquage dans du milieu de culture complet "DMEM" (avec 10% de sérum de veau foetal) en atmosphère humide à 37 °C et 5 % de $CO_2$. Les cellules HacaT ont été ensemencées dans des plaques 24 puits à raison de $1.10^5$ cellules par puits.

Des dérivés sulfoxydes et sulfones de conjugués (Z)-isomères de formule (I) ont été générés par exposition stoechiométrique au peroxyde d'hydrogène ($H_2O_2$), purifiés et enfin quantifiés.

Dans chacun des puits ont été ajoutés 500 µl des sous-produits d'oxydation sulfoxydes et sulfones dérivés des conjugués selon l'invention, à la concentration de 2,5 mM dans du PBS. Après élimination du milieu, la viabilité cellulaire des kératinocytes HaCaT a été mesurée via la "méthode au MTT" ou bromure de 3-(4,5-diméthylthiazol-2-yl)-2,5-diphenyltétrazolium (solution à 500 µg/ml) et par spectrophotométrie (absorbance à 550 nm). Les résultats, rassemblant les valeurs moyennes obtenues à partir de trois expérimentations indépendantes, sont présentés dans le tableau 2 ci-après, en comparaison avec ceux obtenus pour des cellules non irradiées.

Tableau 2

| Composé | % viabilité cellulaire |
|---|---|
| Témoin (cellules non irradiées) | 100 |
| (*Z*)-*N*-(2-hydroxyéthyl)-3-(méthylthiosulfinyl)propénamide (2,5mM) | 94,3 |
| (*Z*)-*N*-(2-hydroxyéthyl)-3-(méthylthiosulfonyl)propénamide (2,5mM) | 93,1 |
| (*Z*)-*N*-(4-hydroxyphénéthyl)-3-(méthylthiosulfinyl)propénamide (2,5mM) | 95,6 |
| (*Z*)-*N*-(4-hydroxyphénéthyl)-3-(méthylthiosulfonyl)propénamide (2,5mM) | 95,0 |

Les résultats du tableau 2 témoignent de l'absence de toxicité des sous-produits d'oxydation des conjugués (Z)-isomères de formule (I).

Test 3 : mise en évidence de la capacité des conjugués (Z)-isomères de formule (I) à viabiliser une lignée cellulaire de kératinocytes soumis à des doses cytotoxiques d'UV-B

Expérimentalement, l'étude a également été réalisée sur une lignée de kératinocytes immortalisés "HaCaT", ensemencée dans des plaques 24 puits à raison de 8000 cellules par centimètres carrés, soit 15200 cellules par puits dans 0,5 ml du milieu de culture (avec 10% de sérum de veau foetal).

Dans chacun des puits ont été ajoutés 500 µl de conjugué selon l'invention, à la concentration de 0,05 à 0,5 mM dans du PBS. Les cellules en présence d'actif ont alors été exposées à des radiations ultra-violettes (UV-B) à la dose modérée de 100 $mJ.cm^{-2}$, puis remises en milieu sans actif pendant 24h. Après élimination du milieu, la viabilité cellulaire des kératinocytes HaCaT a également été mesurée via la "méthode au MTT" (solution à 250 µg/ml) et par spectrophotométrie (absorbance à 570 nm). Les résultats, rassemblant les valeurs moyennes obtenues à partir de trois expérimentations indépendantes, sont présentés dans le tableau 3 ci-après, en comparaison avec ceux obtenus pour deux témoins, des cellules non irradiées et des cellules irradiées mais non traitées avec les conjugués selon l'invention.

Tableau 3

| Composé | % viabilité cellulaire |
|---|---|
| Témoin (cellules non irradiées) | 100 |
| Témoin + irradiation UV-B = 100 mJ/cm | 46 |

(suite)

| Composé | % viabilité cellulaire |
|---|---|
| (*Z*)-*N*-(2-hydroxyéthyl)-3-(méthylthio)propénamide (0,1mM) | 64 |
| (*Z*)-*N*-(2-hydroxyéthyl)-3-(méthylthio)propénamide (0,25mM) | 85 |
| (*Z*)-*N*-(2-hydroxyéthyl)-3-(méthylthio)propénamide (0,5mM) | 94 |
| (*Z*)-*N*-(4-hydroxyphénéthyl)-3-(méthylthio)propénamide (0,1mM) | 70 |
| (*Z*)-*N*-(4-hydroxyphénéthyl)-3-(méthylthio)propénamide (0,25mM) | 93 |
| (*Z*)-*N*-(4-hydroxyphénéthyl)-3-(méthylthio)propénamide (0,5mM) | 100 |
| (*Z*)-*N*-éthyl-3-(méthylthio)propénamide (0,5mM) | 86 |

Les résultats du tableau 3 soulignent, pour les composés selon l'invention, une viabilité cellulaire dose-dépendante, avec une photoprotection des kératinocytes dès 0,1 mM et une photoprotection complète ou quasi-complète à 0,5 mM.

Test 4 : mise en évidence de la capacité des conjugués (Z)-isomères de formule (I) à absorber le rayonnement ultraviolet par photo-isomérisation

Principe : il s'agit de vérifier si les conjugués selon l'invention sont capables de s'isomériser sous irradiation UV-B.

Expérimentalement, 2 mL d'une solution de squalène à 5g/100 mL et 2 mL d'une solution de composé selon l'invention à la concentration de 25 mM dans du méthanol ont été introduites dans une éprouvette graduée de 20 mL, puis complétés à 20 mL avec du méthanol. La préparation ainsi obtenue a été versée dans une boîte de Pétri, sur laquelle a été appliquée, dans un four UV-B, une dose d'irradiation de 1 $J.cm^{-2}$. En fin d'irradiation, les solutions ont été récupérées puis analysées par dosage HPLC. Les volumes ont été préalablement normalisés avec du méthanol.
Des profils chromatographiques ont ainsi été obtenus, et il a été recherché la formation d'isomère E. On a déterminé alors un taux d'isomérisation par comparaison avec un profil témoin.
Les résultats obtenus sont rassemblés dans le tableau 4 ci-après :

Tableau 4

| Composé | % isomérisation |
|---|---|
| (Z)-*N*-(2-hydroxyéthyl)-3-(méthylthio)propénamide (2,5mM) | 62 |
| (Z)-*N*-(4-hydroxyphénéthyl)-3-(méthylthio)propénamide (2,5mM) | 95 |
| (*Z*)-*N*-éthyl-3-(méthylthio)propénamide (2,5mM) | 60 |
| (Z)-*N*-(2-hydroxyéthyl)-3-(méthylthiosulfinyl)propénamide (2,5mM) | 64 |

Les résultats du tableau 4 soulignent que les composés selon l'invention sont capables d'absorber efficacement le rayonnement avec la production de leur stéréoisomère de configuration trans.

Test 5 : mise en évidence de la capacité des conjugués (Z)-isomères de formule (I) à s'opposer à l'isomérisation photo-induite de l'acide *trans*-urocanique

Principe : il s'agit de vérifier si les conjugués selon l'invention sont capables de s'opposer à la photo-isomérisation de l'acide trans-urocanique. Expérimentalement, l'étude a été réalisée de manière quasi-identique aux tests 1 et 4 ci-avant, avec au préalable la vérification que dans les conditions expérimentales définies, l'acide trans-urocanique générait une quantité significative d'acide cis-urocanique que l'on peut mesurer par analyse HPLC.
La dose d'irradiation (1D) a été de 31 $mJ.cm^{-2}$ pour des concentrations en composé de 5 mM. Les résultats obtenus sont rassemblés dans le tableau 5 suivant :

Tableau 5

| Composé | % inhibition de l'acide trans- urocanique |
|---|---|
| (Z)-*N*-(2-hydroxyéthyl)-3-(méthylthio)propénamide (5 mM) | 75 |
| (Z)-*N*-(4-hydroxyphénéthyl)-3-(méthylthio)propénamide (5 mM) | 78 |

Les résultats du tableau 5 soulignent que les composés selon l'invention sont efficaces dans la protection de l'acide trans-urocanique vis-à-vis de l'irradiation UV-B à dose faible ou modérée.

## Revendications

1. Composé dérivé de l'acide (Z)-3-(alkylthio)propénoïque, **caractérisé en ce qu'**il est représenté par la formule générale (I) suivante :

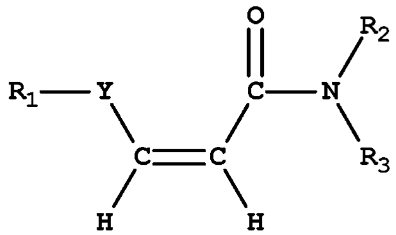

(I)

dans laquelle :

- Y = S ou SO ;
- $R_1$ est un radical alkyle en $C_1$-$C_4$, linéaire ou ramifié ;
- $R_2$ est un atome d'hydrogène ; un radical alkyle en $C_1$-$C_4$, linéaire ou ramifié ;
- $R_3$ est :

  • un radical alkyle en $C_1$-$C_{18}$, un radical alcène en $C_3$-$C_{18}$, un radical alcyne en $C_3$-$C_4$, ledit radical alkyle, alcène ou alcyne étant linéaire ou ramifié, et optionnellement substitué par un radical hydroxyle ou par un groupe hydroxyphényle ou dihydroxyphényle ;
  • la chaîne latérale d'un acide aminé choisi parmi la tyrosine, l'hydroxyproline, la sérine et ses analogues homosérine et isosérine, la thréonine et ses analogues homothréonine et isothréonine ;

à l'exception des composés 2-cis-entadamide A, isopenangine, N-éthyl-cis-3-butylmercaptoacrylamide et N-ethyl-cis-3-méthylmercaptoacrylamide.

2. Composé selon la revendication 1, **caractérisé en ce qu'**il est représenté par la formule générale (II) suivante :

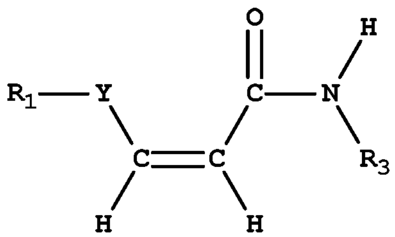

(II)

dans laquelle :

- Y = S ou SO ;
- $R_1$ est un radical alkyle en $C_1$-$C_4$, linéaire ou ramifié ;
- $R_3$ est un radical alkyle en $C_1$-$C_4$, un radical alcène en $C_3$-$C_4$, un radical alcyne en $C_3$-$C_4$, ledit radical alkyle, alcène ou alcyne étant linéaire ou ramifié, et optionnellement substitué par un radical hydroxyle ou par un

groupe hydroxyphényle ou dihydroxyphényle.

**3.** Composé selon la revendication 1 ou 2, **caractérisé en ce que** Y est S.

**4.** Composé selon l'une quelconque des revendications 1 à 3, **caractérisé en ce que** $R_1$ est un radical méthyle.

**5.** Composé selon l'une quelconque des revendications 1 à 4, **caractérisé en ce que** $R_3$ est un radical éthyle substitué par un radical hydroxyle ou par un groupe hydroxyphényle ou dihydroxyphényle.

**6.** Composé selon l'une quelconque des revendications 1 à 5, **caractérisé en ce qu'**il s'agit du (*Z*)-*N*-(4-hydroxyphénéthyl)-3-(méthylthio)propénamide.

**7.** Composition cosmétique ou dermocosmétique, destinée à la photoprotection, **caractérisée en ce qu'**elle comprend, en association avec tout adjuvant physiologiquement compatible avec la peau ou les phanères, à titre d'ingrédient(s) actif(s) principal(aux), un composé de formule générale (IIIa), un composé de formule (IIIb) ou un mélange, de préférence racémique, de ces composés :

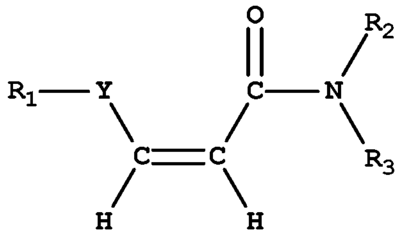

(IIIa)

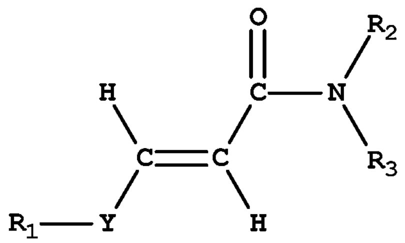

(IIIb)

dans lesquelles :

- Y = S ou SO ;
- $R_1$ est un radical alkyle en $C_1$-$C_4$, linéaire ou ramifié ;
- $R_2$ est un atome d'hydrogène ; un radical alkyle en $C_1$-$C_4$, linéaire ou ramifié ;
- $R_3$ est :
  - un radical alkyle en $C_1$-$C_{18}$, un radical alcène en $C_3$-$C_{18}$, un radical alcyne en $C_3$-$C_4$, ledit radical alkyle, alcène ou alcyne étant linéaire ou ramifié, et optionnellement substitué par un radical hydroxyle ou par un groupe hydroxyphényle ou dihydroxyphényle ;
  - la chaîne latérale d'un acide aminé choisi parmi la tyrosine, l'hydroxyproline, la sérine et ses analogues homosérine et isosérine, la thréonine et ses analogues homothréonine et isothréonine.

**8.** Composition cosmétique ou dermocosmétique selon la revendication 7, qui comprend à titre d'ingrédient(s) actif(s) principal(aux), un composé de formule générale (IVa), un composé de formule (IVb) ou un mélange, de préférence racémique, de ces composés:

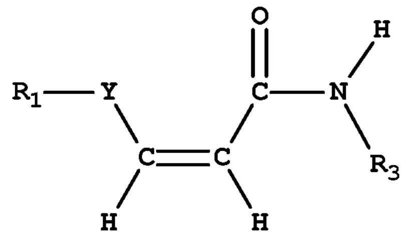

(IVa)

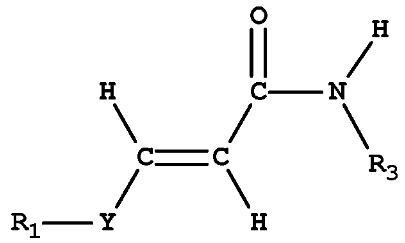

(IVb)

.

dans lesquelles :

- Y = S ou SO ;
- $R_1$ est un radical alkyle en $C_1$-$C_4$, linéaire ou ramifié ;
- $R_3$ est un radical alkyle en $C_1$-$C_4$, linéaire ou ramifié, optionnellement substitué par un radical hydroxyle ou par un groupe hydroxyphényle ou dihydroxyphényle.

**9.** Composition cosmétique ou dermocosmétique selon la revendication 7 ou 8, **caractérisée en ce que** Y, $R_1$ et $R_3$ sont tels que définis dans les revendications 3 à 5.

**10.** Composition cosmétique ou dermocosmétique selon l'une quelconque des revendications 7 à 9, **caractérisée en ce qu'**elle comprend, à titre d'ingrédient actif principal, le (Z)-*N*-(2-hydroxyéthyl)-3-(méthylthio)propénamide ou le (*Z*)-*N*-(4-hydroxyphénéthyl)-3-(méthylthio)propénamide.

**11.** Composition cosmétique ou dermocosmétique selon l'une quelconque des revendications 7 à 10, **caractérisée en ce que** la quantité dudit ingrédient actif principal est comprise entre 0,001 % et 0,1 % en poids par rapport au poids total de la composition.

**12.** Composition cosmétique ou dermocosmétique selon l'une quelconque des revendications 7 à 11, **caractérisée en ce qu'**elle comprend en outre un filtre solaire organique ou inorganique actifs dans l'UV-A et/ou l'UV-B, choisi parmi les anthranilates, les dérivés cinnamiques, les dérivés de dibenzoylméthane, les dérivés salicyliques, les dérivés du camphre, les dérivés de triazine, les dérivés de la benzophénone, les dérivés de beta,beta-diphénylacrylate, les dérivés de benzotriazole, les dérivés de benzalmalonate, les dérivés de benzimidazole, les imidazolines, les dérivés bis-benzoazolyle, les dérivés de benzoxazole, les dérivés de l'acide p-aminobenzoique (PABA), les dérivés de méthylène bis-(hydroxyphényl benzotriazole), les polymères filtres et silicones filtres, les dimères dérivés d'alpha-alkylstyrène, les 4,4-diarylbutadiènes, des pigments ou des nanopigments d'oxydes métalliques (oxydes de titane, de zinc, de fer, de zirconium, de cérium), enrobés ou non, et leurs mélanges.

**13.** Composition cosmétique ou dermocosmétique selon l'une quelconque des revendications 7 à 12, **caractérisée en ce qu'**elle comprend en outre un ou plusieurs actifs choisis parmi les agents déglycants, les agents stimulant la synthèse de collagène ou d'élastine ou prévenant leur dégradation, les agents stimulant la synthèse de glycosaminoglycanes ou de protéoglycanes ou prévenant leur dégradation, les agents augmentant la prolifération cellulaire, les agents dépigmentants ou pro-pigmentants, les agents antioxydants ou anti-radicalaires ou anti-pollution, les agents hydratants, les agents stimulant la lipolyse, les agents drainants ou détoxifiants, les agents anti-inflammatoires, les agents accélérateurs de pénétration, les agents desquamants, les agents apaisants et/ou anti-irritants, les agents astringents, les agents agissant sur la microcirculation, et leurs mélanges.

**14.** Utilisation, dans une composition cosmétique ou dermocosmétique, d'un composé de formule (IIIa) ou d'un composé de formule (IIIb) tels que définis dans la revendication 7, ou d'un mélange de ces composés, comme agent photoprotecteur du rayonnement solaire.

**15.** Composé de formule (IIIa) ou de formule (IIIb) tel que défini dans la revendication 7, ou un mélange de ces composés, pour son utilisation, dans une composition cosmétique ou dermocosmétique, pour la photoprotection contre le rayonnement solaire.

**16.** Utilisation selon la revendication 14 ou composé pour l'utilisation selon la revendication 15, **caractérisé en ce que** ledit composé de formule (IIIa) est le (Z)-*N*-(2-hydroxyéthyl)-3-(méthylthio)propénamide ou le (Z)-*N*-(4-hydroxyphénéthyl)-3-(méthylthio)propénamide.

## Patentansprüche

**1.** Von (Z)-3-(Alkylthio)-Propensäure abgeleitete Verbindung, **dadurch gekennzeichnet, dass** sie durch die folgende allgemeine Formel (I) dargestellt wird:

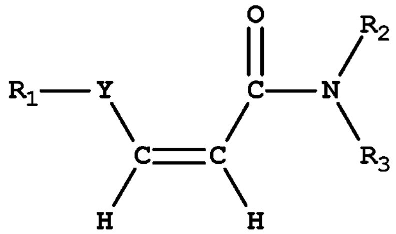

(I)

wobei:

- Y = S oder SO,
- $R_1$ ein linearer oder verzweigter $C_1$-$C_4$-Alkylrest ist,
- $R_2$ ein Wasserstoffatom, ein linearer oder verzweigter $C_1$-$C_4$-Alkylrest ist,
- $R_3$ ist:

  • ein $C_1$-$C_{18}$-Alkylrest, ein $C_3$-$C_{18}$-Alkenrest, ein $C_3$-$C_4$-Alkinrest, wobei der Alkyl-, Alken- oder Alkinrest linear oder verzweigt ist und gegebenenfalls durch einen Hydroxylrest oder eine Hydroxyphenyl- oder Dihydroxyphenylgruppe substituiert ist,
  • die Seitenkette einer Aminosäure, ausgewählt aus Tyrosin, Hydroxyprolin, Serin und seinen Homoserin- und Isoserin-Analogen, Threonin und seinen Homothreonin- und Isothreonin-Analogen,

mit Ausnahme der Verbindungen 2-cis-Entadamid A, Isopenangin, N-Ethyl-cis-3-Butylmercaptoacrylamid und N-Ethyl-cis-3-Methylmercaptoacrylamid.

**2.** Verbindung gemäß Anspruch 1, **dadurch gekennzeichnet, dass** sie durch die folgende allgemeine Formel (II) dargestellt wird:

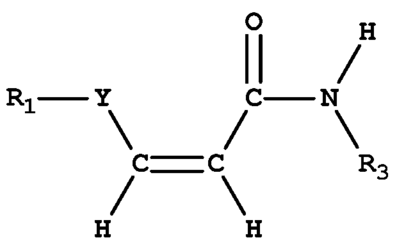

(II)

wobei:

- Y = S oder SO,
- $R_1$ ein linearer oder verzweigter $C_1$-$C_4$-Alkylrest ist,
- $R_3$ ein $C_1$-$C_4$-Alkylrest, ein $C_3$-$C_4$-Alkenrest, ein $C_3$-$C_4$-Alkinrest ist, wobei der Alkyl-, Alken- oder Alkinrest

linear oder verzweigt ist und gegebenenfalls durch einen Hydroxylrest oder eine Hydroxyphenyl- oder Dihydroxyphenylgruppe substituiert ist.

3. Verbindung gemäß Anspruch 1 oder 2, **dadurch gekennzeichnet, dass** es sich bei Y um S handelt.

4. Verbindung gemäß einem der Ansprüche 1 bis 3, **dadurch gekennzeichnet, dass** es sich bei $R_1$ um einen Methylrest handelt.

5. Verbindung gemäß einem der Ansprüche 1 bis 4, **dadurch gekennzeichnet, dass** es sich bei $R_3$ um einen Ethylrest handelt, der durch einen Hydroxylrest oder durch eine Hydroxyphenyl- oder Dihydroxyphenylgruppe substituiert ist.

6. Verbindung gemäß einem der Ansprüche 1 bis 5, **dadurch gekennzeichnet, dass** es sich um (Z)-*N*-(4-Hydroxyphenethyl)-3-(Methylthio)-Propenamid handelt.

7. Kosmetische oder dermokosmetische Zusammensetzung, die für den Lichtschutz bestimmt ist, **dadurch gekennzeichnet, dass** sie in Kombination mit allen Zusatzstoffen, die physiologisch mit der Haut oder den Hautanhangsgebilden verträglich sind, als Hauptwirkstoff(e) eine Verbindung der allgemeinen Formel (IIIa), eine Verbindung der Formel (IIIb) oder eine Mischung dieser Verbindungen, vorzugsweise racemisch, enthält:

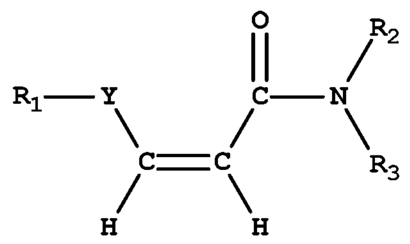

(IIIa)

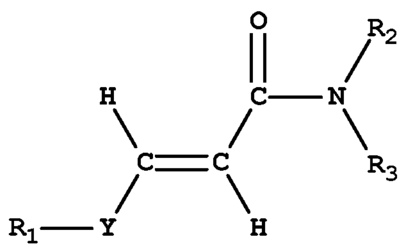

(IIIb)

wobei:

- Y = S oder SO,
- $R_1$ ein linearer oder verzweigter $C_1$-$C_4$-Alkylrest ist,
- $R_2$ ein Wasserstoffatom, ein linearer oder verzweigter $C_1$-$C_4$-Alkylrest ist,
- $R_3$ ist:

  • ein $C_1$-$C_{18}$-Alkylrest, ein $C_3$-$C_{18}$-Alkenrest, ein $C_3$-$C_4$-Alkinrest, wobei der Alkyl-, Alken- oder Alkinrest linear oder verzweigt ist und gegebenenfalls durch einen Hydroxylrest oder eine Hydroxyphenyl- oder Dihydroxyphenylgruppe substituiert ist,
  • die Seitenkette einer Aminosäure, ausgewählt aus Tyrosin, Hydroxyprolin, Serin und seinen Homoserin- und Isoserin-Analogen, Threonin und seinen Homothreonin- und Isothreonin-Analogen.

8. Kosmetische oder dermokosmetische Zusammensetzung gemäß Anspruch 7, die als Hauptwirkstoff(e) eine Verbindung der allgemeinen Formel (IVa), eine Verbindung der Formel (IVb) oder eine Mischung dieser Verbindungen, vorzugsweise racemisch, enthält:

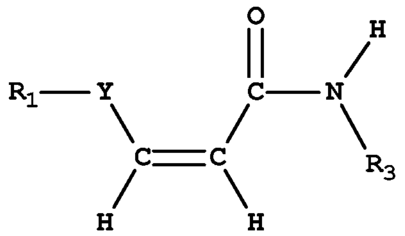

(IVa)

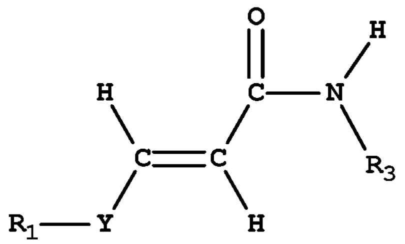

(IVb)

wobei:

- Y = S oder SO,
- $R_1$ ein linearer oder verzweigter $C_1$-$C_4$-Alkylrest ist,
- $R_3$ ein linearer oder verzweigter $C_1$-$C_4$-Alkylrest ist, gegebenenfalls durch einen Hydroxylrest oder eine Hydroxyphenyl- oder Dihydroxyphenylgruppe substituiert ist.

**9.** Kosmetische oder dermokosmetische Zusammensetzung gemäß Anspruch 7 oder 8, **dadurch gekennzeichnet, dass** Y, $R_1$ und $R_3$ wie in den Ansprüchen 3 bis 5 definiert sind.

**10.** Kosmetische oder dermokosmetische Zusammensetzung gemäß einem der Ansprüche 7 bis 9, **dadurch gekennzeichnet, dass** sie als Hauptwirkstoff (Z)-N-(2-Hydroxyethyl)-3-(Methylthio)-Propenamid oder (Z)-*N*-(4-Hydroxyphenethyl)-3-(Methylthio)-Propenamid enthält.

**11.** Kosmetische oder dermokosmetische Zusammensetzung gemäß einem der Ansprüche 7 bis 10, **dadurch gekennzeichnet, dass** die Menge des Hauptwirkstoffs zwischen 0,001 und 0,1 Gew.-%, bezogen auf das Gesamtgewicht der Zusammensetzung, liegt.

**12.** Kosmetische oder dermokosmetische Zusammensetzung gemäß einem der Ansprüche 7 bis 11, **dadurch gekennzeichnet, dass** sie ferner einen organischen oder anorganischen Sonnenschutzfilter umfasst, der in UV-A und/oder UV-B aktiv ist, ausgewählt aus Anthranilaten, Zimtderivaten, Dibenzoylmethan-Derivaten, Salicylderivaten, Kampferderivaten, Triazinderivaten, Benzophenonderivaten, Beta-, Beta-Diphenylacrylat-Derivaten, Benzotriazol-Derivaten, Benzalmalonat-Derivaten, Benzimidazol-Derivaten, Imidazolinen, Bis-Benzoazolyl-Derivaten, Benzoxazol-Derivaten, p-Aminobenzoesäure-Derivaten (PABA), Methylen-bis-(Hydroxyphenylbenzotriazol)-Derivaten, Polymerfiltern und Filtersilikonen, von alpha-Alkylstyrol abgeleiteten Dimeren, 4,4-Diarylbutadienen, Pigmenten oder Nanopigmenten von Metalloxiden (Oxide von Titan, Zink, Eisen, Zirkonium, Cer), die beschichtet oder unbeschichtet sind, und Mischungen davon.

**13.** Kosmetische oder dermokosmetische Zusammensetzung gemäß einem der Ansprüche 7 bis 12, **dadurch gekennzeichnet, dass** sie ferner einen oder mehrere Wirkstoffe enthält, die ausgewählt sind aus Deglykationsmitteln, Mitteln, die die Synthese von Kollagen oder Elastin stimulieren oder deren Abbau vorbeugen, Mitteln, die die Synthese von Glykosaminoglykanen oder Proteoglykanen stimulieren oder deren Abbau vorbeugen, zellproliferationsfördernden Mitteln, depigmentierenden oder pro-pigmentierenden Mitteln, Antioxidantien oder Anti-Radikal- oder Anti-Verschmutzungsmitteln, feuchtigkeitsspendenden Mitteln, Lipolyse stimulierenden Mitteln, Drainage- oder Entgiftungsmitteln, entzündungshemmenden Mitteln, Penetrationsbeschleunigern, Peelingmitteln, beruhigenden und/oder reizlindernden Mitteln, adstringierenden Mitteln, Mitteln, die auf die Mikrozirkulation wirken, und Mischungen davon.

**14.** Verwendung einer Verbindung der Formel (IIIa) oder einer Verbindung der Formel (IIIb), wie in Anspruch 7 definiert, oder einer Mischung dieser Verbindungen in einer kosmetischen oder dermokosmetischen Zusammensetzung als Lichtschutzmittel bei Sonneneinstrahlung.

**15.** Verbindung der Formel (IIIa) oder Formel (IIIb), wie in Anspruch 7 definiert, oder Mischung dieser Verbindungen zur Verwendung in einer kosmetischen oder dermokosmetischen Zusammensetzung zum Lichtschutz gegen Sonnenstrahlung.

**16.** Verwendung gemäß Anspruch 14 oder Verbindung zur Verwendung gemäß Anspruch 15, **dadurch gekennzeichnet, dass** es sich bei der Verbindung der Formel (IIIa) um (Z)-*N*-(2-Hydroxyethyl)-3-(Methylthio)-Propenamid oder (Z)-*N*-(4-Hydroxyphenethyl)-3-(Methylthio)-Propenamid handelt.

## Claims

**1.** A compound derived from (Z)-3-(alkylthio)propenoic acid, **characterized in that** it is represented by the following general formula (I):

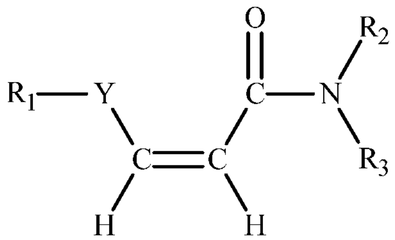

(I)

wherein:

- Y = S or SO;
- $R_1$ is a linear or branched $C_1$-$C_4$ alkyl group;
- $R_2$ is a hydrogen atom, or a linear or branched $C_1$-$C_4$ alkyl group;
- $R_3$ is:
  - a $C_1$-$C_{18}$ alkyl group, a $C_3$-$C_{18}$ alkene group, or a $C_3$-$C_4$ alkyne group, said alkyl, alkene and alkyne groups being linear or branched and optionally substituted with a hydroxyl, a hydroxyphenyl or a dihydroxyphenyl group; or
  - the side chain of an amino acid selected from the group consisting of tyrosine, hydroxyproline, serine and its analogs homoserine and isoserine, threonine and its analogs homothreonine and isothreonine ;

provided that said compound is not one of the following compounds: 2-*cis*-entadamide A, isopenangin, N-ethyl-*cis*-3-butylmercaptoacrylamide and N-ethyl-*cis*-3-methylmercaptoacrylamide.

**2.** The compound according to claim 1, **characterized in that** it is represented by the following general formula (II):

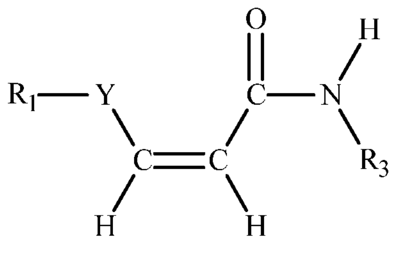

(II)

wherein:

- Y = S or SO;
- $R_1$ is a linear or branched $C_1$-$C_4$ alkyl group;
- $R_3$ is a $C_1$-$C_4$ alkyl group, a $C_3$-$C_4$ alkene group, or a $C_3$-$C_4$ alkyne group, said alkyl, alkene and alkyne groups being linear or branched and optionally substituted with a hydroxyl, a hydroxyphenyl or dihydroxyphenyl group.

**3.** The compound according to claim 1 or 2, **characterized in that** Y is S.

**4.** The compound according to any one of claims 1 to 3, **characterized in that** $R_1$ is a methyl group.

**5.** The compound according to any one of claims 1 to 4, **characterized in that** $R_3$ is an ethyl group substituted by a hydroxyl, a hydroxyphenyl or a dihydroxyphenyl group.

**6.** The compound according to any one of claims 1 to 5, **characterized in that** it is (*Z*)-N-(4-hydroxyphenethyl)-3-(methylthio)propenamide.

**7.** A cosmetic or dermocosmetic composition intended for photoprotection, **characterized in that** it comprises as main active ingredient(s) a compound of formula (IIIa), a compound of formula (IIIb), or a mixture, preferably a racemic mixture, thereof, in combination with any additive physiologically compatible with skin or appendages:

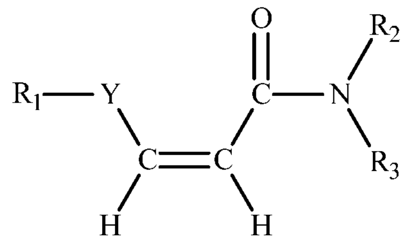

(IIIa)

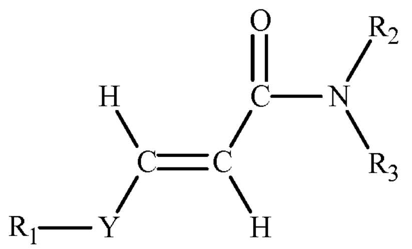

(IIIb)

wherein:

- Y = S or SO;
- $R_1$ is a linear or branched $C_1$-$C_4$ alkyl group;
- $R_2$ is a hydrogen atom, or a linear or branched $C_1$-$C_4$ alkyl group;
- $R_3$ is:

  • a $C_1$-$C_{18}$ alkyl group, a $C_3$-$C_{18}$ alkene group, or a $C_3$-$C_4$ alkyne group, said alkyl, alkene or alkyne groups being linear or branched and optionally substituted with a hydroxyl, a hydroxyphenyl or a dihydroxyphenyl group; or
  • the side chain of an amino acid selected from the group consisting of tyrosine, hydroxyproline, serine and its analogs homoserine and isoserine, threonine and its analogs homothreonine and isothreonine.

**8.** The cosmetic or dermocosmetic composition according to claim 7, **characterized in that** it comprises, as main active ingredient(s), a compound of formula (IVa), a compound of formula (IVb), or a mixture, preferably a racemic mixture, thereof:

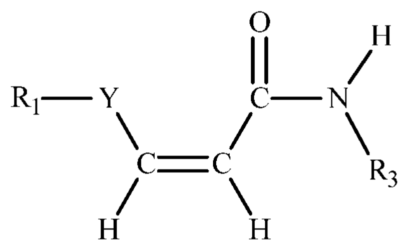

(IVa)

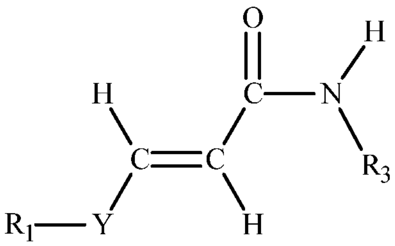

(IVb)

wherein:

- Y = S or SO;
- $R_1$ is a linear or branched $C_1$-$C_4$ alkyl group;
- $R_3$ is a linear or branched $C_1$-$C_4$ alkyl group optionally substituted with a hydroxyl, a hydroxyphenyl or a dihydroxyphenyl group.

**9.** The cosmetic or dermocosmetic composition according to claim 7 or 8, **characterized in that** Y, $R_1$ and $R_3$ are as defined in claims 3 to 5.

**10.** The cosmetic or dermocosmetic composition according to any one of claims 7 to 9, **characterized in that** it comprises as main active ingredient (*Z*)-N-(2-hydroxyethyl)-3-(methylthio)propenamide or (*Z*)-N-(4-hydroxyphenethyl)-3-(methylthio)propenamide.

**11.** The cosmetic or dermocosmetic composition according to any one of claims 7 to 10, **characterized in that** the amount of said main active ingredient is from 0.001% to 0.1% by weight based on the total weight of the composition.

**12.** The cosmetic or dermocosmetic composition according to any one of claims 7 to 11, **characterized in that** it further comprises an organic or inorganic sun filter that is active in the UV-A and/or UV-B, selected from: anthranilates, cinnamic derivatives, dibenzoylmethane derivatives, salicylic derivatives, camphor derivatives, triazine derivatives, benzophenone derivatives, beta,beta-diphenylacrylate derivatives, benzotriazole derivatives, benzalmalonate derivatives, benzimidazole derivatives, imidazolines, bis-benzoazolyl derivatives, benzoxazole derivatives, p-aminobenzoic acid (PABA) derivatives, methylene bis-(hydroxyphenyl benzotriazole) derivatives, filter polymers, filter silicones, dimers derived from alpha-alkylstyrene, 4,4-diarylbutadienes, metal oxide pigments or nanopigments (oxides of titanium, zinc, iron, zirconium, cerium), coated or uncoated, and mixtures thereof.

**13.** The cosmetic or dermocosmetic composition according to any one of claims 7 to 12, **characterized in that** it further comprises one or several active ingredients selected from: deglycation agents, agents that stimulate the synthesis of collagen or elastin or prevent their degradation, agents that stimulate the synthesis of glycosaminoglycans or proteoglycans or prevent their degradation, agents that increase cell proliferation, depigmenting or pro-pigmenting agents, antioxidant or anti-radical or anti-pollution agents, moisturizing agents, agents that stimulate lipolysis, draining or detoxifying agents, anti-inflammatory agents, penetration enhancer agents, desquamative agents, soothing and/or anti-irritating agents, astringent agents, agents that act on the microcirculation, and mixtures thereof.

**14.** Use, in a cosmetic or dermocosmetic composition, of a compound of formula (IIIa) or of a compound of formula (IIIb) as defined in claim 7, or of a mixture of these compounds, as a photoprotecting agent against sun radiation.

**15.** A compound of formula (IIIa) or a compound of formula (IIIb) as defined in claim 7, or of a mixture of these compounds, for use, in a cosmetic or dermocosmetic composition, for the photoprotection against sun radiation.

**16.** Use according to claim 14 or compound for the use of claim 15, **characterized in that** said compound of formula (IIIa) is (*Z*)-N-(2-hydroxyethyl)-3-(methylthio)propenamide or (*Z*)-N-(4-hydroxyphenethyl)-3-(methylthio)propenamide.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 5464832 A
- EP 0410726 A
- US 5300672 A
- US 3914301 A


### Littérature non-brevet citée dans la description


- **ICHIHASHI M. et al.** *Toxicology,* 2003, vol. 189, 21-39
- **BARRESI C. et al.** *J. Invest. Dermatol.,* 2011, vol. 131, 188-194
- **LACOUR et al.** *Annales de Dermatologie et Vénéréologie,* 2007, vol. 134, 18-24
- **RAI R. et al.** *Indian J. Dermatol.,* 2012, vol. 57 (5), 335-342
- **BRUGÈ et al.** *Plos One,* 2014, vol. 9, e83401
- **UTER W. et al.** *Contact Dermatitis,* 2014, vol. 71, 162-9
- **MATSUI M.S. et al.** *J. Invest. Dermatol.,* 2009, vol. 14, 56-59
- **LIEBEL F. et al.** *J. Invest. Dermatol.,* 2012, vol. 132, 1901-1907
- **SCHROEDER P. et al.** *Exp. Gerontol.,* 2008, vol. 43, 629-632
- **IKEGAMI F. et al.** *Chem. Pharm. Bull.,* 1989, vol. 37, 1932-1933
- **FARIA DE MEDEIROS E. et al.** *J. Chem. Soc. Perkin Trans I,* 1991, 27525-2730
- **MC LOONE P. et al.** *J. Invest. Dermatol.,* 2005, vol. 124, 1071-1074
- **GIBBS N.K. et al.** *J. Invest. Dermatol.,* 2011, vol. 131, 14-17
- **POON T.S.C. et al.** *J. Invest. Dermatol.,* 2003, vol. 121, 184-190
- **WOLF P. et al.** *J. Invest. Dermatol.,* 2003, vol. 121, 1080-1087
- **BECK I. et al.** *Int. J. Cosmetic Science,* 1981, vol. 3, 139-152
- **HURKS H.M.H et al.** *J. Invest. Dermatol.,* 1997, vol. 109, 699-703
- **FILLAY-JONES J.J. et al.** *Mutation Res.,* 1998, vol. 422, 155-159